# FASCICULE DE BREVET EUROPEEN

(11) **EP 1 051 255 B1**
(45) Date de publication et mention de la délivrance du brevet: **22.09.2004**
(21) Numéro de dépôt: 98963500.8
(22) Date de dépôt: 23.11.1998
(51) Int. Cl.: B01J 37/02, B01J 29/04, B01J 35/04, B01J 29/06, C07D 301/10

(54) **CATALYSEUR A BASE DE ZEOLITE, SON UTILISATION ET PROCEDE D'EPOXYDATION EN PRESENCE DE CE CATALYSEUR**
ZEOLITHISCHE KATALYSATOR, SEINE VERWENDUNG UND EPOXIDIERUNGSVERFAHREN IN GEGENWART DIESES KATALYSATOR
CATALYST BASED ON ZEOLITE, USE AND EPOXIDATION METHOD IN THE PRESENCE OF SAID CATALYST

(30) Priorité: 01.12.1997 BE 9700973
(43) Date de publication de la demande: 15.11.2000
(73) Titulaire: SOLVAY (Société Anonyme), 1050 Bruxelles (BE)
(72) Inventeur: CATINAT, Jean-Pierre, B-7131 Waudrez (BE); STREBELLE, Michel, B-1150 Bruxelles (BE)
(74) Mandataire: Vande Gucht, Anne
(86) Numéro de dépôt international: PCT/EP1998/007530
(87) Numéro de publication internationale: WO 1999/028035

(56) Documents cités:
- EP-A- 0 526 896
- EP-A- 0 559 021
- DE-A- 4 240 693
- DE-A- 4 334 981
- DE-A- 4 425 672

## Description

L'invention concerne un nouveau catalyseur supporté à base de zéolite au titane. Elle concerne également un procédé de fabrication d'un tel catalyseur ainsi que son utilisation dans des réactions chimiques en phase liquide, comme la réaction d'époxydation d'oléfines sous l'action du peroxyde d'hydrogène. DE-A-4240693 et DE-A 4425672 décrivent des catalyseurs zéolithiques au titane.

Il est connu d'utiliser une zéolite comme catalyseur dans des réactions d'époxydation d'oléfines. Ainsi la demande de brevet EP 1001 19 divulgue un procédé d'époxydation d'oléfines au moyen de peroxyde d'hydrogène en présence d'une zéolite synthétique contenant des atomes de titane. Cette zéolite synthétique contenant des atomes de titane est connue sous le nom de silicalite de titane et abrévié par TS-1.

Il est également connu que les zéolites se désactivent à l'usage et qu'il faut, dès lors, procéder régulièrement à des traitements de régénération généralement par lavage avec des solvants ou par chauffage pour en restituer l'activité. Il est important que la zéolite ne se dégrade pas dans les conditions de régénération.

Il est également connu que les zéolites, et en particulier le TS-1 obtenu selon le procédé décrit dans la demande de brevet mentionnée ci-dessus, peuvent être constituées de particules très fines qu'il est malaisé de séparer du mélange réactionnel pour en effectuer la régénération. Par ailleurs, lorsque les zéolites, et en particulier le TS-1, sont constituées de gros grains, on observe une diminution significative de l'activité catalytique de ce catalyseur d'une part et une mauvaise résistance à l'attrition des particules d'autre part.

L'invention vise à remédier à ces inconvénients en fournissant un nouveau catalyseur de forme telle qu'il soit facile à séparer du mélange réactionnel pour en effectuer la régénération et qui présente de bonnes propriétés mécaniques et une activité élevée.

L'invention a pour objet un catalyseur comprenant une zéolite au titane déposée par imprégnation sur un support en forme de nids d'abeilles tel que décrit dans la revendication 1. Par "forme de nids d'abeilles, on entend désigner une forme constituée d'éléments à structure alvéolaire, quelle que soit la forme des alvéoles. Le catalyseur selon l'invention possède un niveau d'activité catalytique proche de celui d'une poudre fine et peut être régénéré sans perte notable de zéolite ou de l'activité catalytique. Plus particulièrement, il a été trouvé que le dépôt d'une zéolite sur un support en forme de nids d'abeilles permet d'atteindre un niveau d'activité très élevé, comparable au niveau d'activité de la poudre fine sans toutefois en avoir les inconvénients.

Avantageusement, le support en forme de nids d'abeilles est constitué d'un matériau inerte, supportant les conditions de régénération et sur lequel il est possible d'y faire adhérer la zéolite au moyen d'un liant. Comme support, les silices conviennent bien. Il peut par exemple s'agir de silices combinées à d'autres oxydes de magnésium, d'aluminium et leurs mélanges. Le support est de préférence de la cordiérite ou de la mullite. Une préférence particulière est montrée pour la cordiérite car elle conduit à une meilleure adhérence de la zéolite au support. Lorsque le catalyseur est ultérieurement régénéré en présence d'un agent oxydant tel que le peroxyde d'hydrogène, une préférence est montrée pour la mullite car elle résiste mieux à de telles conditions de régénération qui conduisent à une acidification du milieu. Toutefois, la cordiérite peut également être régénérée en présence d'un agent oxydant à condition de maintenir le pH, lors de la régénération, à une valeur de 3 à 4 environ.

Le support en forme de nids d'abeilles se présente généralement sous la forme d'une cartouche comprenant de 10 à 1200 cellules par inch² (cpi²). De préférence, le nombre de cellules est de 50 à 450 cpi², par exemple de 70 à 400 cpi².

Par zéolite, on entend désigner un solide contenant de la silice qui présente une structure cristalline microporeuse. La zéolite est avantageusement exempte d'aluminium. La zéotite contient du titane. De préférence, la zéolite selon l'invention est une zéolite dans laquelle plusieurs atomes de silicium ont été remplacés par des atomes de titane.

De bons résultats ont été obtenus avec des zéolites de type silicalite de titane. Ceux-ci présentent avantageusement une structure cristalline de type ZSM-5, ZSM-11, MCM-41 ou de type bêta. Elles présentent de préférence une bande d'absorption infrarouge à environ 950-960 cm⁻¹. Celles répondant à la formule xTiO₂(1-x)SiO₂ dans laquelle x est de 0,0001 à 0.5, de préférence de 0,001 à 0,05 sont performantes. Des matériaux de ce type, connus sous le nom de TS-1, présentent une structure zéolitique cristalline microporeuse analogue à celle de la zéolite ZSM-5. Les propriétés et les principales applications de ces composés sont connues (B. Notari; Structure-Activity and Selectivity Relationship in Heterogeneous Catalysis, R.K. Grasselli and A. W. Sleight Editors; Elsevier; 1991; p. 243-256). Leur synthèse a été étudiée notamment par A. Van der Poel et J. Van Hooff (Applied Catalysis A, 1992; Volume 92, pages 93-111) et par Thangaraj et al.; Zeolites 12 (1992), 943-950).

La teneur en zéolite dans le catalyseur selon l'invention exprimée en pourcentage en poids de zéolite dans le catalyseur est généralement supérieure ou égale à 1 % et inférieure ou égale à 60 %. De préférence, la teneur en zéolite est supérieure ou égale à 5 % et inférieure ou égale à 40 %.

Le catalyseur selon l'invention résiste, lors des phases de conditionnement du catalyseur ou de régénération, à des chauffages à sec (à 500 °C par exemple) ou en présence de solvant sans perte significative d'éléments actifs. En outre, le peroxyde d'hydrogène, utilisé comme réactif oxydant lors de la synthèse ou comme agent de régénération, n'engendre pas non plus de dommage notable au catalyseur.

L'invention concerne également un procédé de fabrication du catalyseur décrit ci-dessus. Selon ce procédé, la zéolite est, dans une première étape, dispersée dans un liant et la dispersion ainsi obtenue est déposée par imprégnation sur un support en forme de nids d'abeilles dans une deuxième étape.

Généralement, le liant mis en oeuvre est un composé à base de silicium. On peut citer à titre d'exemples les silices colloïdales, les sols de silice, les silicates (par exemple les tétraalkyl silicates) et les résines siliconées. Les silices colloïdales sont préférées. Plusieurs qualités de silice colloïdale peuvent convenir. Elles sont caractérisées notamment par la taille des particules, par leur surface spécifique, par leur pH et par la nature du contre-ion.

Généralement, la taille des particules de silice colloïdale est comprise entre 1 et 30 µm. De préférence, la taille des particules est comprise entre 5 et 25 µm. Une préférence est marquée pour des particules dont la taille est comprise entre 7 et 20 µm.

Des particules de zéolite trop fines entraînent une thixotropie gênante. Des particules trop grosses conduisent à une sédimentation trop rapide pour une mise en oeuvre efficace. Généralement, la taille des particules est supérieure ou égale à 0,1 µm et inférieure ou égale à 10 µm. Avantageusement, la taille des particules est supérieur ou égale à 1 µm et inférieure ou égale à 5 µm.

Les silices colloïdales peuvent présenter un pH acide ou basique. Lorsque le pH est acide, le contr'ion est avantageusement un anion chlorure et/ou les particules de silice peuvent se couvrir en surface d'un couche d'alumine. Lorsque le pH est alcalin, le contr'ion est avantageusement un cation sodium ou un cation ammonium et/ou des atomes de silicium superficiels peuvent être remplacés par des atomes d'aluminium.

Dans la première étape du procédé selon l'invention, on prépare une dispersion de la zéolite dans un liant, éventuellement sous agitation. Le rapport en poids entre la quantité de zéolite engagée et la quantité de liant peut varier dans une très large proportion. Ce rapport est généralement inférieur ou égal à 20 et supérieur ou égal à 0,1. De préférence, ce rapport est inférieur ou égal à 15 et supérieur ou égal à 0,5. Un rapport proche de 10 permet de limiter la quantité de matière à déposer sur le support en forme de nids d'abeilles tout en fixant la quantité désirée de zéolite sans toutefois boucher les canaux du support.

Il peut être avantageux d'y ajouter de l'eau. Généralement, la quantité d'eau dans la dispersion est telle que la dispersion contient au moins 10 g de zéolite par 100 g d'eau. Avantageusement, la quantité d'eau est telle que la dispersion contient au moins 20 g de zéolite par 100 g d'eau. Cette concentration est généralement inférieure ou égale à 175 g de zéolite par 100 g d'eau. Avantageusement, cette concentration est inférieure à 150 g de zéolite par 100 g d'eau. Une préférence particulière est montrée pour un domaine de concentrations allant de 50 à 90 g de zéolite par 100 g d'eau.

L'imprégnation du support en forme de nids d'abeilles se fait généralement soit en versant la dispersion sur le support en forme de nids d'abeilles, soit en immergeant le support dans la dispersion. Généralement, l'étape d'imprégnation se déroule à la température ambiante. Après l'étape d'imprégnation, le liquide adhérant aux parois des nids d'abeilles, peut être chassé par un jet d'air comprimé pour éviter le bouchage des canaux des nids d'abeilles. L'opération peut éventuellement être répétée. Le support en forme de nids d'abeilles peut ensuite être séché (par exemple à l'air ambiant) pendant une durée variant généralement de quelques minutes à quelques heures, de préférence durant une période comprise entre 30 minutes et 180 minutes. Le support imprégné peut ensuite être séché (par exemple dans une étuve ventilée) à une température supérieure ou égale à 105 °C et inférieure ou égale à 300 °C, de préférence supérieure ou égale à 180 °C et inférieure ou égale à 250 °C. La durée du séchage du support imprégné est généralement supérieure ou égale à 2 heures et inférieure ou égale à 3 jours. De préférence, cette durée est supérieure ou égale à 12 heures et inférieure ou égale à 2 jours.

L'opération d'imprégnation peut être répétée plusieurs fois, éventuellement avec séchage intermédiaire. Les imprégnations successives peuvent être réalisées dans les mêmes conditions que décrites ci-dessus pour déposer plusieurs couches de zéolite sur le support. II est préférable de travailler avec des solutions concentrées en TS-1 pour limiter le nombre d'étapes d'imprégnation pour atteindre une quantité de TS-1 déposée donnée. Toutefois, s'il est généralement avantageux d'utiliser une solution concentrée pour le dépôt de la première couche d'imprégnation, on a constaté qu'il peut être préférable d'utiliser des solutions plus diluées pour les couches d'imprégnation déposées ultérieurement. Au terme de la dernière imprégnation, le catalyseur peut être calciné (par exemple dans un four statique) à une température supérieure ou égale à 200 °C et inférieure ou égale à 800 °C. De préférence, cette température est supérieure ou égale à 300 °C et inférieure ou égale à 600 °C. Avantageusement, cette température est supérieure ou égale à 400 °C et inférieure ou égale à 550 °C. Le temps de calcination est généralement compris entre quelques heures et plusieurs jours, de préférence ce temps de séchage est supérieur ou égal à 6 heures et inférieur ou égal à 12 heures.

La quantité de matière déposée sur le support en forme de nids d'abeilles comprend la quantité de zéolite déposée ainsi que la quantité de liant utilisé. La quantité de matière déposée sur le support en forme de nids d'abeilles dépend principalement de la taille du support. Par exemple, pour des nids comprenant 400 cellules par inch², le gain en poids est généralement supérieur ou égal à 10 % et inférieur ou égal à 80 %. De préférence, le gain en poids est supérieur à 20 % et inférieur ou égal à 70 %. Avantageusement, le gain en poids est supérieur ou égal à 30 % et inférieur ou égal à 55 %.

Généralement, la quantité de catalyseur souhaitée est atteinte par le dépôt de 3 à 5 couches de dispersion sur le support en forme de nids d'abeilles. Pour une même quantité de matière déposée, l'augmentation du nombre de couches conduit à une meilleure adhérence du dépôt au support.

On peut favoriser l'adhérence de la couche de zéolite sur le support en forme de nids d'abeilles par préimprégnation dudit support avec le liant pur. De même, le support peut être prétraité en milieu acide ou basique selon la nature du support pour obtenir un dépôt plus important lors de la première étape d'imprégnation. Un agent tensioactif peut également être ajouté à la dispersion pour augmenter la quantité de matière déposée lors de la première imprégnation.

Le catalyseur selon l'invention peut être régénéré après plusieurs cycles de réaction généralement par traitement thermique, par lavage ou par traitement au moyen d'un agent oxydant tel que le peroxyde d'hydrogène et retrouver ainsi un niveau d'activité et une sélectivité proches du catalyseur frais.

Généralement. le catalyseur selon l'invention peut être utilisé dans des réactions chimiques en phase liquide comme par exemple des réactions d'oxydation ou d'époxydation. Par conséquent, l'invention concerne aussi l'utilisation d'un catalyseur comprenant une zéolite déposée sur un support en forme de nids d'abeilles dans des réactions chimiques en phase liquide.

Le catalyseur selon l'invention convient particulièrement bien pour des réactions d'époaydation d'oléfines par un composé peroxydé en phase liquide. Les produits obtenus sont les époxydes (ou oxirannes) correspondants. Le procédé de la présente invention s'applique à toutes les oléfines, aliphatiques ou alicycliques, quels que soient le nombre d'atomes de carbone dans la chaîne carbonée. la position de l'insaturation et la présence d'une fonction chimique dans la chaine. A titre d'exemple non limitatif d'oléfine utilisable dans le procédé selon la présente invention, on peut citer l'éthylène, le propylène, le chlorure d'allyle, le butène-1, le butène-2, l'octène-1, le cyclohexène, le cyclooctène et l'oxyde de mésityle. Le chlorure d'allyle et le propylène conviennent particulièrement bien pour la synthèse d'épichlorhydrine ou d'oxyde de propylène. Le composé peroxydé est de préférence le peroxyde d'hydrogène.

Un solvant de réaction est habituellement ajouté pour permettre la mise en contact de l'oléfine et du peroxyde d'hydrogène. Parmi les solvants possibles, le méthanol est préféré.

Le catalyseur selon la présente invention est généralement utilisé dans un procédé en continu ou en discontinu. Un procédé en continu est préféré.

La température de réaction est généralement supérieure ou égale à 0 °C et inférieure ou égale à 100 °C. De préférence, cette température est supérieure ou égale à 5 °C et inférieure ou égale à 50 °C. Avantageusement, cette température est supérieure ou égale à 10 °C et inférieure ou égale à 40 °C.

Le temps de séjour est le rapport entre le volume du réacteur vide et le débit d'alimentation. Le temps de séjour par réacteur est généralement supérieur ou égal à 1 minute et inférieur ou égal à 100 minutes. De préférence, le temps de séjour est supérieur ou égal à 5 minutes et inférieur ou égal à 80 minutes. Avantageusement, le temps de séjour est supérieur ou égal à 10 minutes et inférieur ou égal à 50 minutes.

Le rapport molaire entre la quantité d'oléfine engagée et la quantité de peroxyde d'hydrogène est généralement supérieur ou égal à 1 et inférieur ou égal à 20. De préférence, ce rapport est supérieur ou égal à 1,5 et inférieur ou égal à 10.

Le rapport molaire entre la quantité d'oléfine engagée et la quantité de solvant est généralement supérieur ou égal à 2 et inférieur ou égal à 50. De préférence, ce rapport est supérieur ou égal à 5 et inférieur ou égal à 10.

Dans les exemples qui suivent,
- le taux de conversion du peroxyde d'hydrogène est donné par l'équation : TC = 1 - (le nombre de moles de peroxyde d'hydrogène retrouvé divisé par le nombre de moles de peroxyde d'hydrogène mis en oeuvre); il est exprimé en pourcent,
- la sélectivité est donnée par le rapport entre la quantité d'époxyde obtenue divisée par la somme de tous les produits formés,
- la quantité d'époxyde formée est la quantité formée après le temps nécessaire pour que le taux de conversion du peroxyde d'hydrogène après une mise en régime d'une heure, ait chuté à 75 % de cette valeur.

L'invention se trouve plus amplement illustrée dans les exemples non limitatifs suivants.

### Exemple 1

Dans cet exemple, on a préparé un catalyseur comprenant du TS-1 déposé sur un support en forme de nid d'abeilles.

Le tableau I ci-après rassemble les détails de préparation du catalyseur.

**Tableau I**

| | |
|---|---|
| Diamètre des particules de TS-1 | 2,1 µm |
| Nature du liant | sol de silice |
| matériau du support | cordiérite |
| nids d'abeilles mis en oeuvre | 2 cartouches h= 10 cm, Ø = 2.6 cm |
| Nombre de cellules /inch² | 400 |
| poids initial du support (g) | 46,5 |
| concentration en TS-1 dans la dispersion (g/100g H₂O) | 90 (1 ère couche) 50 (2e couche) |
| gain de poids à la 1ère imprégnation (g) | 7,9 |
| gain de poids à la 2ème imprégnation (g) | 6,3 |
| séchage intermédiaire | 18 h à 180 °C |
| calcination | 6 h à 500 °C |
| incrément total de poids du support (g) | 14.2 |
| Rapport TS-1/liant (g) | 51,5/48,6 |

### Exemples 2 à 4

Le catalyseur obtenu dans l' exemple 1 a été utilisé dans des réactions d'époxydation.

L'installation comporte deux réacteurs tubulaires verticaux, thermostatisés. disposés en cascade. Deux cartouches de catalyseur sont placées dans chaque réacteur. Chaque réacteur est muni d'une boucle et d'une pompe permettant la recirculation du liquide sur le catalyseur. Le contrôle de la température est assurée par un serpentin parcouru par une huile régulée en température dans chaque réacteur.

On a disposé dans chaque réacteur de 125 ml muni d'une boucle de recirculation (volume total = 300 ml), 7,3 grammes de TS-1 déposés sur les deux cartouches. Le premier réacteur est alimenté en continu à un débit donné par une solution de chlorure d'allyle et de peroxyde d'hydrogène dans du méthanol (chlorure d'allyle / H₂O₂ = 2 mol / mol; concentration en H₂O₂ de 1,33 mol / kg) à une température de T °C. Le mélange de réactifs (oléfine, peroxyde d'hydrogène et méthanol) est préparé juste avant son introduction à débit constant en tête du premier réacteur.

La vitesse linéaire de passage de la solution en recirculation dans le réacteur a été réglée à 0.94 m / min et le débit de recirculation est de l'ordre de 30 litres /heure.

La durée des essais a été fixée sur base d'une décroissance de 25 % de l'activité initiale du catalyseur dans le premier réacteur après une mise en régime d'une heure.

Dans ces conditions, à une température donnée et pour un temps de séjour défini, le chlorure d'allyle est transformé en épichlorhydrine.

Le tableau II reprend les conditions et les résultats des essais réalisés.

**Tableau II**

| Exple | Conditions | | | Résultats | | |
|---|---|---|---|---|---|---|
| | | | | Sortie du 2ème réacteur | | |
| | Température T (°C) | Temps de séjour /réacteur (min) | Débit d'alimentation (ml/h) | TC (H₂O₂) (%) | Sélectivité en épichlorhydrine | quantité d'épichlorhydrine formée (g) |
| 2 | 40 | 40 | 187 | 99,4 | 89,7 | 238 |
| 3 | 10 | 20 | 375 | 77,0 | 99,1 | 150 |
| 4 | 25 | 50 | 150 | 98,4 | 95,1 | 218 |

D'une manière analogue, le propylène, qui est alors introduit sous forme gazeuse dans le réacteur, est transformé en oxyde de propylène.

## Revendications

1. Catalyseur d'époxydation comprenant une zéolite au titane déposée par imprégnation à l'aide d'un liant sur un support en forme de nids d'abeilles.

2. Catalyseur selon la revendication 1 dans lequel la zéolite au titane est du silicalite de titane.

3. Catalyseur selon la revendication 2 dans lequel la zéolite-au titane est du TS-1.

4. Procédé de fabrication d'un catalyseur d'époxydation comprenant une zéolite au titane déposée par imprégnation à l'aide d'un liant sur un support en forme de nids d'abeilles selon lequel, dans une première'étape, la zéolite au titane' est dispersée dans un liant et la dispersion ainsi obtenue est déposée sur un support en forme de nids d'abeilles dans une deuxième étape.

5. Procédé selon la revendication 4 dans lequel le liant est une silice colloïdale.

6. Procédé selon l'une quelconque des revendications 4 ou 5 dans lequel le rapport en poids entre la quantité de zéolite au titane mise en oeuvre et la quantité de liant mise en oeuvre est inférieur ou égal à 20 et supérieur ou égal à 0,1.

7. Procédé selon l'une quelconque des revendications 4 à 6 dans lequel l'imprégnation du support se fait en plusieurs étapes, éventuellement avec séchage intermédiaire.

8. Procédé selon l'une quelconque des revendications 4 à 7 dans lequel le support est traité par le liant avant l'étape d'imprégnation.

9. Utilisation d'un catalyseur d'époxydation comprenant une zéolite au titane déposée par imprégnation à l'aide d'un liant sur un support en forme de nids d'abeilles dans des réactions chimiques en phase liquide.

10. Utilisation selon la revendication 9 pour la synthèse d'époxydes, de préférence l'épichlorhydrine ou l'oxyde de propylène, par réaction entre une oléfine, de préférence le chlorure d'allyle ou le propylène, et un composé peroxydé, de préférence du peroxyde d'hydrogène.

11. Procédé de fabrication d'époxydes, de préférence l'épichlorhydrine ou l'oxyde de propylène, par réaction entre une oléfine, de préférence le chlorure d'allyle ou le propylène, et un composé peroxydé, de préférence du peroxyde d'hydrogène en présence d'un catalyseur comprenant une zéolite au titane déposée par imprégnation à l'aide d'un liant sur un support en forme de nids d'abeilles.

## Patentansprüche

1. Epoxidationskatalysator, umfassend einen Titanzeolith, der mit Hilfe eines Bindemittels auf einem bienenwabenförmigen Träger durch Imprägnierung abgelagert ist.

2. Katalysator nach Anspruch 1, worin der Titanzeolith ein Titansilicalit ist.

3. Katalysator nach Anspruch 2, worin der Titanzeolith TS-1 ist.

4. Verfahren zur Herstellung eines Epoxidationskatalysators, der einen Titanzeolith umfaßt, der mit Hilfe eines Bindemittels auf einem bienenwabenförmigen Träger durch Imprägnierung abgelagert ist, wonach in einer ersten Stufe der Titanzeolith in einem Bindemittel dispergiert und in einer zweiten Stufe die solcherart erhaltene Dispersion auf einem bienenwabenförmigen Träger abgelagert wird.

5. Verfahren nach Anspruch 4, worin das Bindemittel ein kolloidales Siliciumoxid ist.

6. Verfahren nach einem der Ansprüche 4 oder 5, worin das Gewichtsverhältnis zwischen der eingesetzten Titanzeolithmenge und der eingesetzten Bindemittelmenge unter oder gleich 20 und über oder gleich 0,1 beträgt.

7. Verfahren nach einem der Ansprüche 4 bis 6, worin.das Imprägnieren des Trägers in mehreren Stufen erfolgt, gegebenenfalls mit einem Trocknen dazwischen.

8. Verfahren nach einem der Ansprüche 4 bis 7, worin der Träger vor der Imprägnierstufe mit dem Bindemittel behandelt wird.

9. Verwendung eines Epoxidationskatalysators, der einen Titanzeolith umfaßt, der durch Imprägnieren mit Hilfe eines Bindemittels auf einem bienenwabenförmigen Träger abgelagert worden ist, in chemischen Reaktionen in flüssiger Phase.

10. Verwendung nach Anspruch 9, für die Synthese von Epoxiden, vorzugsweise von Epichlorhydrin oder von Propylenoxid, durch Reaktion zwischen einem Olefin, vorzugsweise Allylchlorid oder Propylen, und einer Peroxidverbindung, vorzugsweise Wasserstoffperoxid.

11. Verfahren zur Herstellung von Epoxiden, vorzugsweise von Epichlorhydrin oder von Propylenoxid, durch Reaktion zwischen einem Olefin, vorzugsweise Allylchlorid oder Propylen, und einer Peroxidverbindung, vorzugsweise Wasserstoffperoxid, in Gegenwart eines Katalysators, der einen Titanzeolith umfaßt, der durch Imprägnieren mit Hilfe eines Bindemittels auf einem bienenwabenförmigen Träger abgelagert worden ist.

## Claims

1. Epoxidation catalyst comprising a titanium containing zeolite deposited by impregnation with the aid of a binder on a honeycomb-shaped support.

2. Catalyst according to Claim 1, in which the titanium containing zeolite is titanium silicalite.

3. Catalyst according to Claim 2, in which the titanium containing zeolite is a TS-1.

4. Process for the manufacture of an epoxidation catalyst comprising a titanium containing zeolite deposited by impregnation with the aid of a binder on a honeycomb-shaped support, according to which, in a first stage, the titanium containing zeolite is dispersed in the binder and, in a second stage, the dispersion thus obtained is deposited on a honeycomb-shaped support.

5. Process according to Claim 4, in which the binder is a colloidal silica.

6. Process according to either one of Claims 4 and 5, in which the ratio by weight of the amount of titanium containing zeolite employed to the amount of binder employed is less than or equal to 20 and greater than or equal to 0.1.

7. Process according to any one of Claims 4 to 6, in which the impregnation of the support is carried out in several stages, optionally with intermediate drying.

8. Process according to any one of Claims 4 to 7, in which the support is treated with the binder before the impregnation stage.

9. Use of a catalyst comprising a titanium containing zeolite deposited by impregnation with the aid of a binder on a honeycomb-shaped support in liquid-phase chemical reactions.

10. Use according to Claim 9 in the synthesis of epoxides, preferably epichlorohydrin or propylene oxide, by reaction between an olefin, preferably allyl chloride or propylene, and a peroxide compound, preferably hydrogen peroxide.

11. Process for the manufacture of epoxides, preferably epichlorohydrin or propylene oxide, by reaction between an olefin, preferably allyl chloride or propylene, and a peroxide compound, preferably hydrogen peroxide, in the presence of a catalyst comprising a titanium containing zeolite deposited by impregnation with the aid of a binder on a honeycomb-shaped support.
